# EUROPEAN PATENT APPLICATION

(11) **EP 4 310 103 A1**
(43) Date of publication of application: **24.01.2024**
(21) Application number: 22770103.4
(22) Date of filing: 18.03.2022
(51) Int. Cl.: C08B 15/00, C12N 9/20, C12N 11/14, C12P 7/62, B82Y 40/00, C08H 8/00, D21C 3/20, C07C 67/02

(54) **FUNCTIONALIZED CELLULOSE, METHOD OF ENZYMATIC FUNCTIONALIZATION OF CELLULOSE, PROCESS OF ENZYMATIC FUNCTIONALIZATION OF CELLULOSE USING AN ORGANIC ACID AND PROCESS FOR THE PRODUCTION OF A CELLULOSE WITH INCREASED HYDROPHOBICITY AND ARTICLE**

(30) Priority: 18.03.2021 BR 102021005126
(71) Applicant: Suzano S.A., 41810-012 Pituba, Salvador, BA (BR); Universidade de São Paulo - USP, 05508-220 São Paulo (BR)
(72) Inventor: SIQUEIRA, Germano, 12240-681 São José dos Campos - SP (BR); ARANTES, Valdeir, 12603-000 Lorena - SP (BR); MAROTTI, Braz De Souza, 12607-070 Lorena - SP (BR)
(74) Representative: Aera A/S
(86) International application number: PCT/BR2022/050097
(87) International publication number: WO 2022/192978

(57) **Abstract**

The present invention relates to a functionalized cellulose, in which the cellulose comprises hydrophobic ester groups from fatty acids, and also to processes and methods of functionalizing cellulose that produce cellulose with increased hydrophobicity.

## Description

### FIELD OF THE INVENTION

The present invention relates to a functionalized cellulose with hydrophobic ester groups from organic acids, as well as its functionalization processes and methods that give cellulose an increased hydrophobic characteristic. The cellulose is preferably a microfibrillated (CMF) or nanofibrillated (CNF) cellulose.

### BACKGROUND OF THE INVENTION

Environmental sustainability takes place with economic development associated with environmental preservation and the application of raw materials from renewable sources such as, for example, sources of lignocellulosic materials, with emphasis on sugar cane and bleached eucalyptus kraft pulp (BEKP). Both cultures have high productivity, extensive distribution, in addition to having an already established and constantly growing market. These materials are sources of macromolecules such as lignin and biopolymers such as cellulose and hemicelluloses, with cellulose being the most prominent due to its numerous applications in multiple industrial segments, such as the pulp and paper sector, packaging and bioenergy, Brazil being the second largest world pulp producer, sending two thirds of its production to the international market. Consequently, this is one of the main raw materials for the elaboration of several new products, including cellulose microparticles and nanoparticles, such as microfibrillated cellulose (CMF) and nanofibrillated cellulose (CNF), respectively.

Cellulose particles are isolated from a highly packed set of fibrils. Therefore, they can be considered as an alternative to more expensive materials, such as fibers and carbon nanotubes. For example, the particles can be isolated through different methods, both physical and chemical, and they have extremely desirable properties for the industrial sector, such as high mechanical resistance, lightness and highly hydrophilic due to the large number of hydroxyls, thus making it an excellent material for the production of composites, in particular, if a nanoparticle, the making of nanocomposites. With the aim of expanding its scope of applications, it is desirable to change its physicochemical properties through the functionalization of cellulose particles, such as microfibrillated cellulose (CMF) or nanofibrillated cellulose (CNF), thus expanding its compatibility with other materials.

The art reveals, as Huang et al. (HUANG, J. et al. Fully Green Cellulose Nanocomposites. In: HANIEH, K. et al. (Ed.). Handbook of Nanocellulose and Cellulose Nanocomposites. 1. ed. Weinheim: Wiley- VCH, 2017. Ip. 301-334.), that the surface of nanocelluloses can be functionalized both by physical interactions (adsorption of molecules and macromolecules) and using an approach that aims to establish covalent bonds between cellulosic materials and the binding agent. Chemical reactions that establish these bonds can be catalyzed by chemical or biochemical routes (enzymes). Basically, all chemical functionalizations have the main purpose of introducing stable negative or positive electrostatic charges on the surface, making it possible to obtain better dispersion or adjust the surface energy characteristics of nanocelluloses to improve compatibility, especially when used in conjunction with nonpolar structures or hydrophobic properties in nanocomposites, as revealed by Habibi, Lucia and Rojas (HABIBI, Y.; LUCIA, L. A.; ROJAS, O. J. Cellulose nanocrystals: chemistry, self-assembly, and applications. Chemical reviews, v. 110, n. 6, p. 3479 - 500, 9 June 2010. Available at: <http://www.ncbi.nlm.nih.gov/pubmed/20201500>).

The art reveals the potential of functionalized nanocelluloses using chemical catalysts, applied to several areas, such as biomedical, textile, pneumatic and paper industry.

Industrially, American Process Incorporation (Atlanta/Georgia, USA) and InoFib (Grenoble/Isere, France) have developed processes for the production of functionalized nanocelluloses. American Process uses a lignin extracted from the lignocellulolytic material itself for the functionalization of cellulose (CNC-L/CNF-L) in a process known as AVAP^{®}. Inofib, a French company founded in 2012, has a portfolio where it is possible to find functionalized or non-functional CNFs (INOFIB. InoFib. Available at: <http://www.inofib.fr/lentreprise/>. Accessed on: 12 mar. 2019). Both companies use only chemical routes for the development of their material.

Regarding biotechnological routes, there are reactions where enzymes are used as catalysts, and have advantages such as mild reaction conditions, reduced energy consumption, in addition to their products being usable in the biomedical area ("drug delivery" and biosensors). Despite these advantages, the use of enzymes as catalysts in functionalization reactions of cellulose nanoparticles is scarce in the literature, especially microfibrillated (CMF) or nanofibrillated (CNF) cellulose, an area that is still practically unexplored, but with incredible commercial potential.

A cellulose with increased hydrophobicity can be achieved by modifying the hydroxyls present on the surface of such celluloses, both on a micro and nano scale, as in a microfibrillated (CMF) or nanofibrillated (CNF) cellulose, with the introduction of more hydrophobic chemical groups, in a process known as functionalization. Enzymatic functionalization, aiming at obtaining artificially designed and structurally controlled nanocelluloses in a single step, has been attracting attention, as reported by Boẑiĉ (BOẐIĈ, M.; GORGIEVA, S.; KOKOL, V. Laccase-mediated functionalization of chitosan by caffeic and gallic acids for modulating antioxidant and antimicrobial properties. Carbohydrate Polymers, v. 87, no. 4, p. 2388-2398, 2012). The formation of esters is a way to endow cellulosic surfaces with a more hydrophobic nature.

The present invention overcomes the problems of the art by applying an enzymatic functionalization that reacts organic acids, preferably fatty acids, and hydroxyl groups of cellulose as a substrate, an esterification reaction thus generating a cellulosic product with increased hydrophobic properties. Despite the efforts of the art, such as the use of the enzyme with acetic anhydride, the present invention employs a new class of chemical compounds, such as organic acids, achieving greater hydrophobicity value. Preferably, the organic acid has a carbon chain of 2 to 18 carbons, preferably fatty acids. Additionally, the process is done with care for the environment and provides environmental sustainability to the process, since it has fewer steps than art and, mainly, organic acids can be obtained from vegetable raw material.

### BRIEF DESCRIPTION OF THE INVENTION

The present invention provides a functionalized cellulose, wherein the cellulose comprises hydrophobic ester groups. Preferably, the functionalized cellulose, a first object of the present invention, has hydrophobic ester groups originating from organic acids, preferably fatty acids. In a particular embodiment, ester groups derived from organic acid are incorporated into a cellulose by the action of an enzyme, the enzyme may be a lipase. The organic acid may be of vegetable origin, and may be selected, among others, from butanoic acid, oleic acid, palmitic acid, myristic acid or mixtures thereof. Methods of enzymatic functionalization of cellulose are also disclosed, in which the functionalization for each 1 g of cellulose takes place with at least 1 M of organic acid in the presence of 1 g of enzyme, in which there is a ratio cellulose: acid: enzyme of 1:50:1.

### BRIEF DESCRIPTION OF THE FIGURES

Figure 1Ais a CMF size analysis performed by laser diffraction performed after solvent switching (A).
Figure 1B is a CMF size analysis performed by laser diffraction after functionalization reactions using different fatty acids (B).
Figure 2 is representative of a correlation of the values in Table 1 with the different contact angles using CMF before and after the functionalization reactions with an illustrative image of the contact angle.
Figure 3A is representative of spectra generated through the XPS technique with analyzes performed using the microfibrillated cellulose (CMF) of Figure 1A, prior to the functionalization reactions.
Figure 3B is representative of spectra generated through the XPS technique with analyzes performed using the microfibrillated cellulose (CMF) from Figure 1B, after using butanoic acid (B) as an acyl donor.
Figure 3C is representative of spectra generated through the XPS technique with analyzes performed using the microfibrillated cellulose (CMF) from Figure 1B, after using oleic acid (C) as an acyl donor.
Figure 4A is representative with an image and spectra generated using the AFM-IR technique of CMF bleached eucalyptus Kraft pulp prior to the functionalization reaction (A).
Figure 4B is representative with an image and spectra generated using the AFM-IR technique of CMF of bleached eucalyptus Kraft pulp obtained after functionalization of cellulose with oleic acid (B) as acyl donor group.
Figure 4C is representative with an image and spectra generated using the AFM-IR CMF technique of bleached eucalyptus Kraft pulp obtained after functionalization of cellulose with butanoic acid (C) as acyl group donor.
Figure 5Ais representative of the cellulose spectrum generated using the NMR technique, prior to the functionalization reaction (A).
Figure 6 is an atomic force microscopy image of bleached Kraft pulp (BEKP) CNF fibers, exemplary pulps used in the present invention. (B).

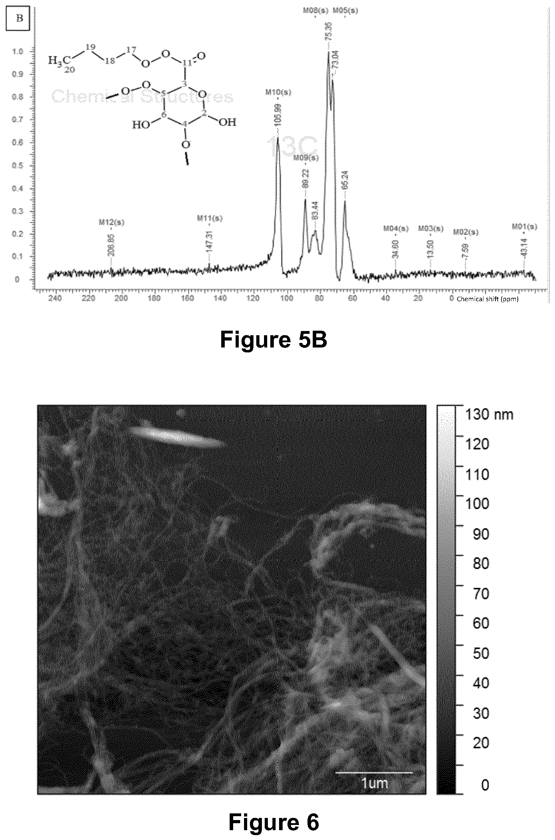

Figure 6 is an atomic force microscopy image of bleached Kraft pulp (BEKP) CNF fibers, exemplary pulps used in the present invention.

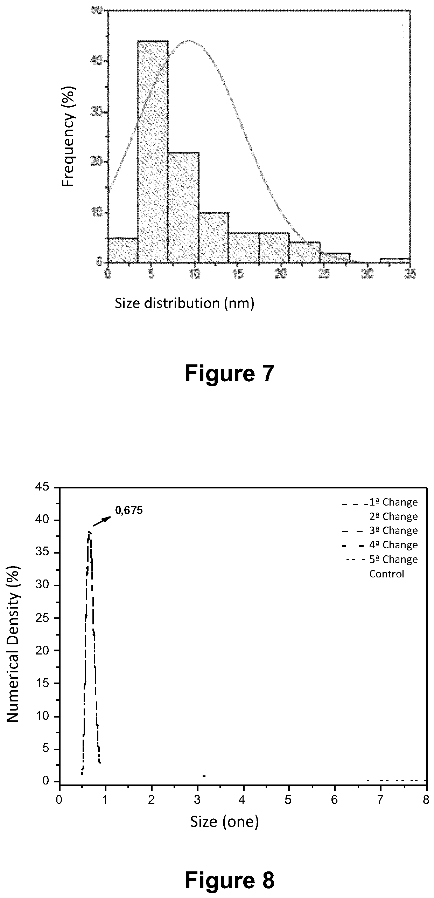

Figure 7 is a histogram of mean diameter distribution of CNF fibers from bleached Kraft pulp (BEKP), exemplary pulps used in the present invention.
Figure 8 is an illustration of the particle size distribution for CNF of bleached sugarcane bagasse pulp during solvent switching.

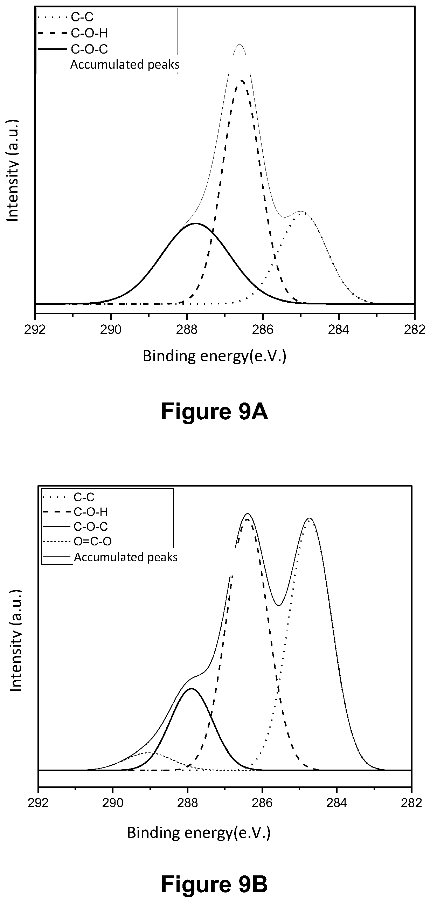

Figure 9A is a representation of Spectra of the bonds present in cellulose generated through the X-ray excited photoelectron spectroscopy (XPS) technique of a Bleached Eucalyptus Kraft Pulp (A) prior to enzymatic functionalization treatment.
Figure 9B is a representation of Spectra of the bonds present in cellulose generated through the X-ray photoelectron spectroscopy (XPS) technique of a Bleached Eucalyptus Kraft Pulp (A) after enzymatic functionalization treatment.

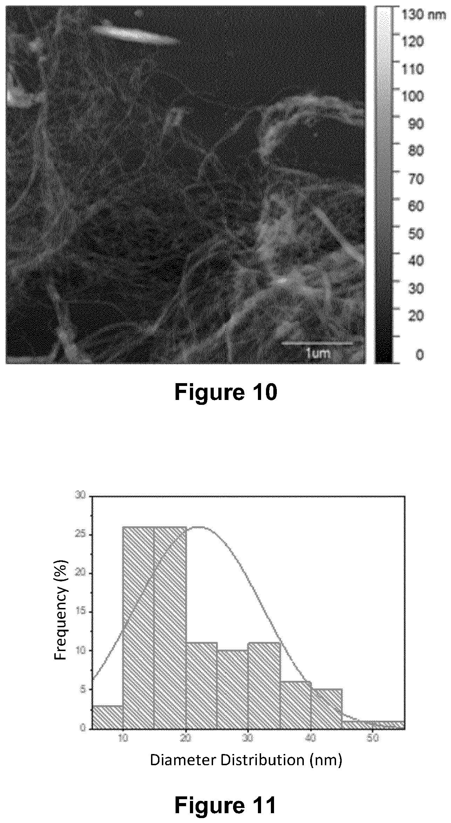

Figure 10 is an Atomic Force Microscopy image for Bleached Sugar Cane Bagasse Pulp CNF fibers.
Figure 11 is a histogram of distribution of the mean diameter of the CNF of Bleached Sugarcane Bagasse.

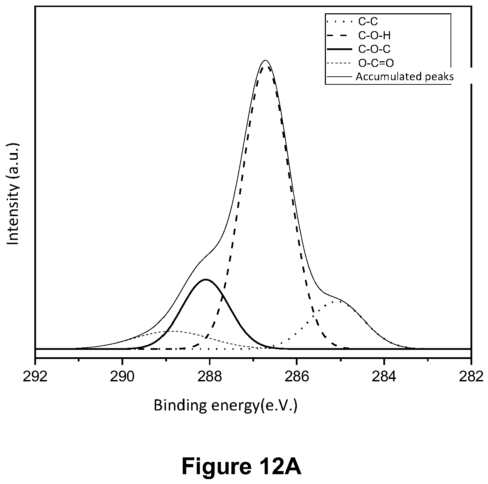

Figure 12A is a representation of Spectra of the bonds present in cellulose generated through the technique of X-ray photoelectron excited spectroscopy (XPS) of a pulp of bleached sugarcane bagasse (A) prior to enzymatic functionalization treatment.

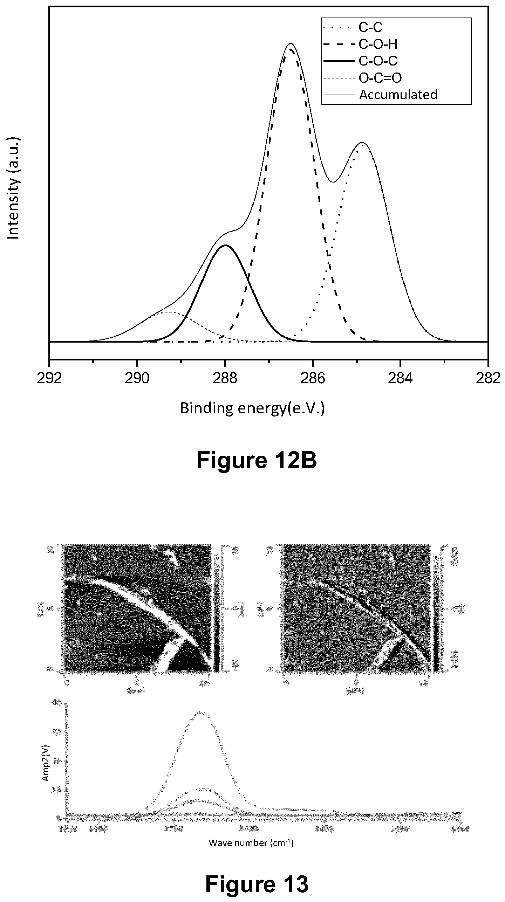

Figure 12B is a representation of Spectra of the bonds present in cellulose generated through the technique of X-ray photoelectron excited spectroscopy (XPS) of a pulp of bleached sugarcane bagasse after the enzymatic functionalization treatment (B).
Figure 13 is an image generated by AFM and its respective FT-IR spectrum. Reaction with immobilized enzyme after recycle number 5 (CFM-F5).

### DETAILED DESCRIPTION OF THE INVENTION

The present invention refers to a functionalized cellulose, processes and methods for obtaining it. The present invention provides the functionalization of a cellulose, which can be cellulose fiber, microfibrillated cellulose, nanofibrillated cellulose, or mixtures thereof. Preferably, the cellulose is a microfibrillated (CMF) or nanofibrillated (CNF) cellulose. For the purposes of the present invention, cellulose refers to those polysaccharides which consist of linear chains of, for example, several hundred to many thousands of D-glucose units with beta (1-4) linkages, and which are present in raw materials of wood. The amounts of cellulose present in the wood and pulp fiber feedstock are measured in accordance with the present invention using the TAPPI T-249 cm-09 method.

In this sense, several sources of lignocellulosic materials can be used, with emphasis on sugarcane bagasse and bleached eucalyptus kraft pulp (BEKP).

In this sense, cellulose fibers have many hydroxyl groups in their structure, which allows easily establishing hydrogen bonds, as well as giving them a hydrophilic character. When microfibrillated or nanofibrillated, this ability to form bonds increases, due to fiber sizes, interweaving and contact surfaces. Therefore, functionalization, especially in comminuted pulp, becomes more important.

In a first embodiment, the present invention provides a functionalized cellulose, wherein the cellulose comprises hydrophobic ester groups. Preferably, the functionalized cellulose, a first object of the present invention, has hydrophobic groups originating from organic acid, in which the organic acid is an acid with a carbonic chain of 2 to 18 carbons, more preferably a fatty acid and, even more preferably, fatty acids selected from butanoic acid, oleic acid, palmitic acid, myristic acid or mixtures thereof, endowed with hydrophobic ester groups.

In a particular embodiment, ester groups derived from organic acid are incorporated into a cellulose by the action of an enzyme. The enzyme may be a lipase.

Lipases are enzymes that can catalyze hydrolysis reactions or synthesis of ester bonds. Many of the lipases that can be used in the present invention are readily available as commercially available products. The lipase used, according to the invention, can be of a single modality or a combination of lipases. Examples of lipase suitable for the present invention are Amano Lipase A, Lipase from porcine pancreas, Lipase from *Candida sp,* antartica and rugosa, Lipase from *Aspergillus oryzae,* Lipase B *Candida antarctica* immobilized on Immobead 150. For example, EC 3.1.1.3 lipases are cited.

The amount of lipase used is preferably from [1.25] to [5] percent by mass, particularly from [2] to [3] percent, based on the mass of the acyl group-donating source for the reaction.

The organic acid may be of vegetable origin. Furthermore, the organic acid is an organic acid with a carbon chain of 2 to 18 carbons, preferably the acid is a fatty acid and, more preferably, the fatty acid can be selected from butanoic acid, oleic acid, palmitic acid, myristic acid or their mixtures. The amount of organic acid used is preferably from 32.5 to 70% by mass, based on the mass of the reaction mixture, particularly from 60 to 65%.

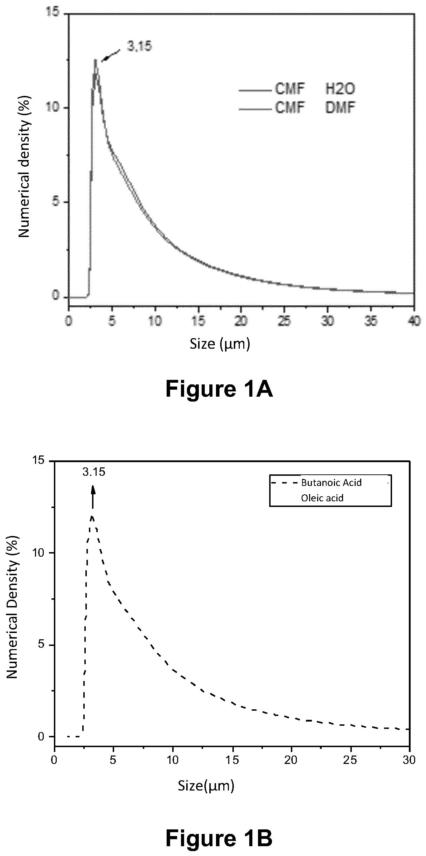

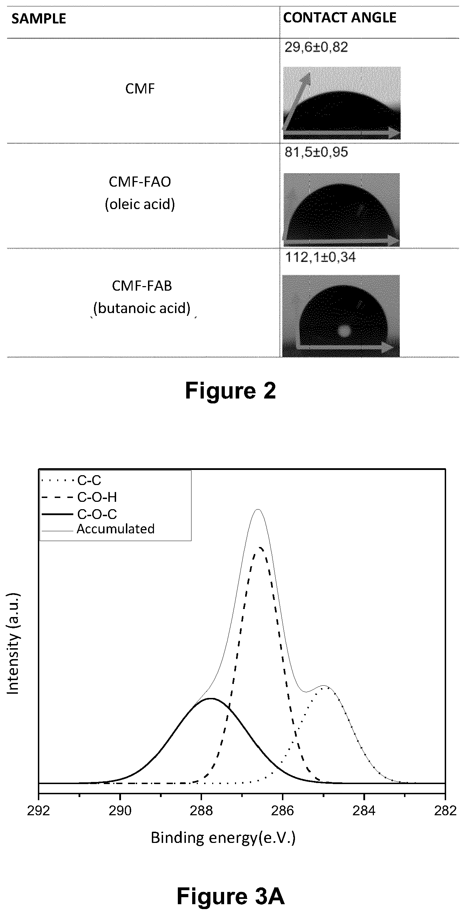

Functionalized cellulose, object of the present invention, advantageously does not show an increase in its dimensions after functionalization. This way, using Figures 1A and 1B in a comparative way, it is possible to verify that the functionalization allows obtaining a cellulose of the same size, despite having its hydrophobicity increased. As illustrated in Figure 12, contact angles obtained by cellulose functionalization are displayed, corroborated by Figures 2A-2C, which illustrates XPS Spectra that indicate an increase in the amount of carbon-carbon bonds coming from the fatty acid incorporated into the CMF.

This way, the functionalized cellulose obtained preferably has an increased amount of ester groups. Preferably, the presence of ester groups is about 50 mmol/g cellulose. In this sense, the functionalized cellulose, object of the present invention, presents a degree of functionalization (substitution) of at least 0.01 and degree of surface substitution of at least 0.01.

A second object of the present invention is to provide a method of enzymatic functionalization of cellulose, the method comprising functionalization for each 1 g of cellulose with at least 1 M of organic acid in the presence of at least 1 g of enzyme, wherein has a cellulose: organic acid: enzyme ratio of 1:50:1. As an example, under excess, for each 1 g of cellulose, 588 g of a fatty acid were used in the presence of 47 g of lipase. In this sense, the present invention also provides a method of esterification of cellulose with an organic acid, achieved by introducing ester groups from a fatty acid into cellulose by enzymatic catalysis of a lipase.

A third object of the present invention is a process for the enzymatic functionalization of cellulose from an organic acid, comprising the following steps:
1. Dispersing a cellulose into an aqueous medium;
2. Replacing aqueous medium with ethanol/acetone mixture;
3. Resuspending the cellulose in dimethylformamide, obtaining a mass concentration of at least 1%;
4. Keeping under agitation, adding, for each 100g of cellulose suspension at 1% m/m in dimethylformamide (DMF), an acid: enzyme mass ratio of at least 50:1, producing a reaction mixture, then letting it rest;
5. Recovering a functionalized cellulose by centrifuging the suspension;
6. Optionally, resuspending the functionalized cellulose in acetone, followed by centrifugation, removing the supernatant and resuspending the cellulose in an aqueous solution of 1% sodium dodecyl sulfate (SDS);
7. Optionally, followed by centrifugation to obtain functionalized cellulose for resuspension in distilled water.

Preferably, step 4 comprises at least 221g of DMF.

As an example of an embodiment, a microfibrillated cellulose (CMF) was diluted in aqueous medium until reaching a concentration of 1% (m/m) and a solvent change was performed using successive centrifugations. Briefly, 10 mL of an aqueous suspension of CMF at 1% m/m were centrifuged for 5 min in a 50 mL conical centrifuge tube at 5 °C and 13,300 g (6,000 rpm); the supernatant was collected and discarded. Then, 10 mL of a mixture of ethanol/acetone (1:1) was added, with homogenization of the mixture by stirring, and the suspension was centrifuged again under the same conditions. This procedure was repeated a total of 5 times. At the end of the fifth centrifugation, the CMF was resuspended in 10 mL DMF, obtaining a final concentration of 1% (m/m) and an aliquot of approximately 1 mL was taken and analyzed for particle size by laser diffraction.

The functionalization reactions took place in 125 mL Erlenmeyer flasks, with a total reaction volume of 60 mL, under orbital stirring at 170 rpm for 48 h. The reaction was conducted using 25g of oleic acid as the acyl donor group, 4.25g of the suspension of 1% m/m CMF in DMF, 33g of DMF, and 2g of lipase (CALB, Sigma). After 48h, the reaction mixture was centrifuged under the conditions mentioned above to remove residual organic acid, and the solid material (CMF) was resuspended in 10 mL of distilled water and heated to boiling point for 5 min in order to deactivate the enzyme. Then, the suspension was centrifuged again and the obtained pellet was initially resuspended in 10 mL of acetone and centrifuged 4 times. After removing the supernatant in the fourth centrifugation step, the CMF was resuspended in 10 mL of 1 % aqueous solution of sodium dodecyl sulfate (SDS) and centrifuged 5 times. Finally, the CMF obtained after the fifth centrifugation was resuspended in 10 mL of distilled water and centrifuged again.

Figure 1A and 1B illustrate the size analyzes performed by laser diffraction with readings taken before (1A) and after the solvent change (1B) and also performed after the functionalization reactions using different fatty acids (1B). Figures 1A and 1B clearly demonstrate that there was no change in CMF sizes during all functionalization steps, analyzes carried out in laser diffraction equipment. As verified, there is no change in the size of the particles evaluated in the particle analyzer, and the esterification reactions occurred in the CMF without an increase in size due to the change of solvent performed.

The CMF obtained after these steps was used for particle size analysis, atomic force microscopy-AFM, AFM-IR, NMR, contact angle and X-ray excited photoelectron spectroscopy - XPS.

Contact angle analyzes were performed using the Rame-Hart 300F1 goniometer. The equipment was used to measure the contact angle with drops of 1 µL of deionized water on a film prepared with CMF and functionalized CMF samples (CMF-Fs). The films were prepared by drying a diluted solution (0.1%) of functionalized CMF/CMF for 24 h at room temperature. To allow good statistical control of the measurement, 3 drops were placed in each sample and 5 angular measurements were taken in each drop. Values for the contact angle of the control CMF were close to 30° due to its hydrophilicity, these results being similar to those found in the literature. Initially, it is possible to notice that the biochemical route used for functionalization demonstrates a positive result, when compared to non-functionalized CMF (control). Different organic acids were compared in the functionalization reactions, being the oleic and butanoic acid that presented a high hydrophobicity when compared to the control. Butanoic acid reached high values and reached hydrophobic values (> 110°) unlike oleic acid (> 80°), as can be seen in Table 1 of Figure 2.

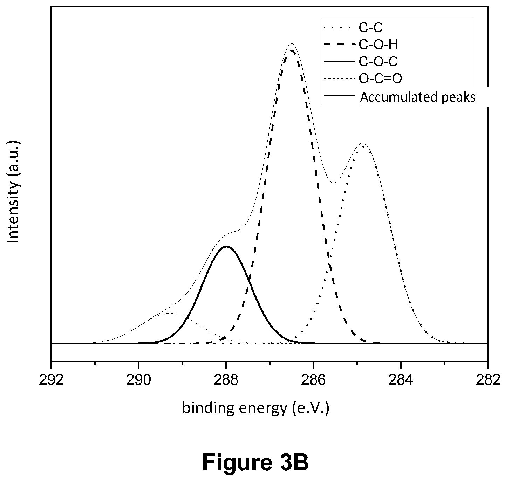

The surface modification of the CMF was further analyzed by X-ray photoelectron spectroscopy (XPS). The analysis was performed using a commercial spectrometer (Thermo Scientific K-Alpha) equipped with a Mg K line (h = 1253.6 eV) at low pressure and operated at 10 eV. As can be seen by comparing Figures 3A, and 3B, the results indicate that there were clear changes in the chemical composition of the surface, increasing the number of carbon-carbon bonds.

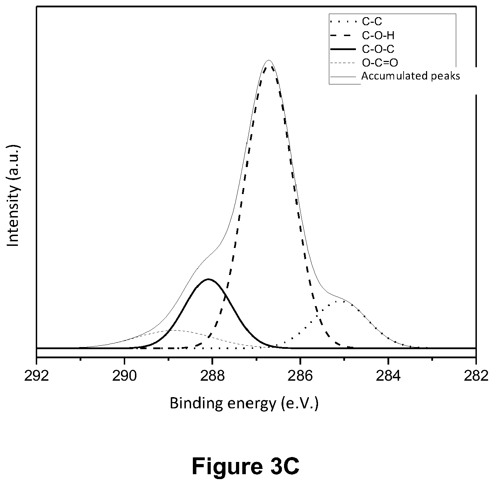

Specifically, Figures 3A, 3B and 3C show Spectra generated using the XPS technique. The analyzes were carried out using CMF before the functionalization reactions (A), and after using butanoic acid (B) and oleic acid (C) as acyl donors. Analyzing the Figure, it is possible to verify an increase in the amount of carbon-carbon bonds coming from the organic acid incorporated to CMF (B and C).

Furthermore, when analyzing the proportions of the carbon atom before and after the functionalization, according to Table 2, below, an increase in the amount of carbon atoms present on the surface is verified, these atoms coming from the oleic acid linked to the CMF polymer.

**Table 2: Proportion of carbon atoms and their relation to oxygen atoms found on the surface of films from CMF derived from Bleached Kraft Pulp, before (control, CMF-H2O and CMF-DMF) and after enzymatic esterification reactions (CMF- FAB and CMF-FAO).**

| SAMPLE | CARBON (%) | NITROGEN (%) | LINK TO OXYGEN / CARBON |
|---|---|---|---|
| CMF (H₂O) | 59.07±0.31 | - | 0.67 |
| CMF (DMF) | 59.35±0.20 | - | 0.69 |
| CMF-FAB | 64.05±0.45 | 0.29±0.04 | 0.54 |
| CMF-FAO | 63.25±0.19 | 2.86±0.31 | 0.53 |

Wherein CMF-FAB is a butanoic acid functionalized microfibrillated cellulose and CMF-FAO is an oleic acid functionalized microfibrillated cellulose.

Specifically, Table 2 demonstrates the proportion of oxygen atoms and their relation to the carbon atoms obtained by XPS, found on the surface of films from different lignocellulosic raw materials, before (control) and after the enzymatic esterification reactions: Observing the table it is possible to conclude that there was an increase in the amount of carbon in the CMFs, thus decreasing the oxygen/carbon ratio after the enzymatic esterification reactions.

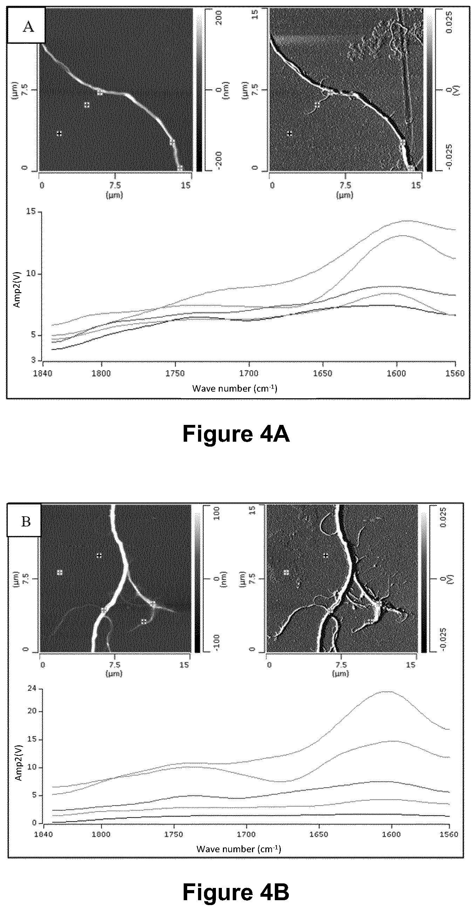

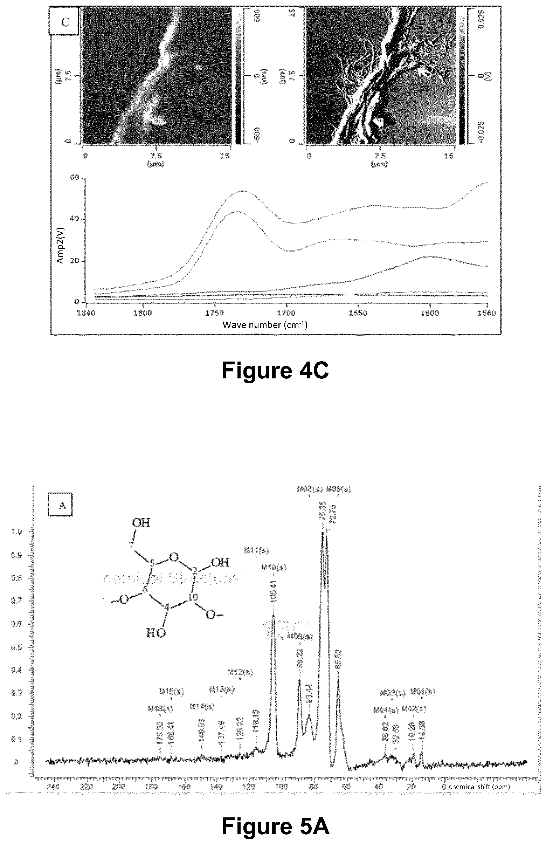

As illustrated in Figures 4A-4C, another qualitative analysis technique was performed using AFM coupled with FT-IR, a technique known as AFM - IR. This technique allows that, in addition to the generation of images, infrared spectra are generated from a specific point on the microfiber. The results indicate that the esterification reaction occurred satisfactorily using both butanoic and oleic acids for the esterification of CMF, evidenced by the appearance of a characteristic band of ester groups close to 1740 cm-1 (Figures 4B and 4C), unlike of non-functionalized CMF (Figure 4A). Furthermore, the image generated by AFM indicates that the CMF is the same size, even after the functionalization reactions. Both analyzes were carried out at the National Nanotechnology Laboratory (LNNano) located at the National Center for Research in Energy and Materials (CNPEM).

Figure 5 illustrates Nuclear Magnetic Resonance (NMR) Spectra generated using the samples, before (Figure 5A) and after (Figure 5B) butanoic acid functionalization reactions (Figure 5). The analyzes were carried out in the Avance III 400 Equipment (Bruker) - 9.4 Tesla (400 MHz for hydrogen frequency) equipped with a probe: 4 mm CP/MAS. The generated spectra were analyzed using the ACD Spectrus Processor 2018 software version 2.5 and the peaks found are listed in Table 3.

**Table 3: Peaks obtained through NMR spectra.**

| Chemical Shift (ppm) | Control (not functionalized) | Functionalized - butanoic acid |
|---|---|---|
| 14.08-36.52 | - | (CH₃, CH₂ (sp² and sp³) aliphatic) |
| 65.24-105.99 | (CH ring near OH, COC) | (CH ring near OH, COC) |
| 175.35 | - | (C=O) carbonyl |

Specifically, Table 3 demonstrates the main chemical groups and their respective "chemical shifts" values found in the NMR spectra shown in Figure 5A and Figure 5B. After the functionalization reactions, new types of carbon hybridization (sp3 and sp2) and new chemical groups (carboxylic) are found. This chemical change shown in the spectra is potentially originated from the esterification reaction of the hydroxyls present in CMF with butanoic acid, catalyzed by the enzyme lipase.

The analysis of the spectra suggests the presence of carbon atoms found in the spectra in the pyranoside rings and demonstrate that there were chemical changes in the CMF after the functionalization reactions (Figures 5A and 5B). The appearance of peaks with values varying between 14.08 and 36.62 ppm (Figure 5B) indicates the presence of aliphatic carbon atoms derived from butanoic acid. Furthermore, it is possible to verify a small peak with a value of 175 ppm, indicating the presence of the carboxylic group derived from the esterification of cellulose with acid. Therefore, this analysis corroborates the statement that the functionalization reaction was able to generate ester groups in CMF from butanoic acid.

When physical and chemical analysis methodologies are used together, it is possible to analyze characteristics that are impossible when these techniques are used in isolation. Therefore, when using NMR, it is proven that the CMFs were effectively functionalized, that is, esterified using organic acid as an acyl donor group through enzymatic catalysis. In addition, through the XPS and contact angle, it is evident that the physicochemical characteristics of the surface of the films generated using this reaction were modified, presenting characteristics such as hydrophobicity and presence of ester groups, nonexistent in the control material (prior to the reaction).

This way, as described, an object of the present invention is a process of enzymatic functionalization of cellulose starting from an organic acid in which step (1), of dispersing cellulose in aqueous medium, is at a proportion of at least 0.25% by mass of cellulose.

Besides, the process of enzymatic functionalization of cellulose from an organic acid, object of the present invention, presents a mixture of ethanol/acetone of step (2) within a ratio of 1:5 to 5:1. Preferably, the process comprises a rest as in step (4) of at least 12h, preferably 48h. Still, step (4) can be followed by a step (4a) in which the reaction mixture is centrifuged to remove the residual organic acid, as described. After step (4a) of the functionalization process, a step (4b) can take place in which the enzyme is deactivated by heating.

Additionally, the present invention provides a method for producing a cellulose with increased hydrophobicity, comprising:
(a) supplying a cellulosic raw material;
(b) in the presence of a lipase, functionalizing said cellulosic raw material, by introducing ester groups derived from an organic acid, to generate a functionalized cellulose with increased hydrophobicity;
(c) recovering said functionalized cellulose with increased hydrophobicity.

Example 2 demonstrates the enzymatic functionalization of nanocellulose isolated from bleached eucalyptus kraft pulp (BEKP). In this example, a bleached eucalyptus kraft pulp (BEKP) was initially suspended at 1% cellulose solids (m/m) and processed in a disk ultra-refiner (SuperMassColloider, Masuko, Model MKCA6-5J), using water as a solvent. The MKGA10-80 model discs are made of ceramic material (aluminium oxide and resin) without porosity, preventing any nanometric particle infiltration and allowing a better fit between the discs. One of the disks remained stationary and the other in motion, rotating at 1,600 rpm. The distance between the discs was determined and fixed. Briefly, the zero position of movement was determined by means of the noise generated by the disks touching each other. From the zero-movement position, the ultra-refiner was fed with the cellulose suspension and the discs were immediately adjusted to the position -100 µm of amplitude between discs. Although the position is negative, the presence of the cellulose suspension ensures that the discs do not touch each other and that the discs do not wear out.

The CNF suspension sample taken right after processing (energy consumption of 21kWh/kg) was diluted to a concentration of approximately 0.01% (m/v) and dripped onto the mica support. The mica with the samples was placed in the desiccator for at least 4 hours. After sample preparation, images were taken using the Nanosurf FlexAFM microscope (Switzerland) and FMR silica probe (Nanoworld) at the National Nanotechnology Laboratory (LNnano) of the Surface Sciences Laboratory, National Center for Research in Energy and Matter (CNPEM, Campinas, Brazil). The system was operated using the intermittent contact technique, at a nominal resonant frequency of 75 kHz and a nominal force constant (spring) of 2.8 m/m. The results indicate that under the conditions used, it was possible to produce CNF with extremely small diameters and lengths greater than 1 µm, as can be seen in Figure 6. From the images treated in the Gwyddion 2.49 Software program (64 bits), 100 diameter measurements of CNF particles were taken and plotted on a graph in the Origin software (Version 2019) to analyze the diameter class and the average diameter, displayed in the Histogram of Figure 7.

After CNF preparation, the CNF solvent was exchanged in aqueous suspension for an organic solvent using successive centrifugations. Briefly, 10 mL of the CNF aqueous suspension (1% m/m) in a 50 mL conical centrifuge tube was centrifuged for 5 min at 5 °C and 13,300 g (10,000 rpm), and the supernatant was collected and discarded. Then, 10 mL of the ethanol/acetone mixture (1:1) was added, the mixture was homogenized by manually shaking the tube and the suspension was centrifuged again under the same conditions. This procedure was repeated a total of 5 times. After each centrifugation, an aliquot of approximately 1mL was taken and analyzed for particle size by laser diffraction. At the end of the fifth centrifugation, the CNF was resuspended in 10 mL of dimethylformamide (DMF), obtaining a final concentration of 1% (m/m). As expected, there was no change in the size of the particles evaluated in the particle analyzer, as can be seen in Figure 8. Therefore, it was possible to confirm that the esterification reactions occurred in the CNF without a significant change in size due to the change of solvent performed.

The functionalization reactions took place in 125 mL Erlenmeyer flasks, with a total reaction volume of 60 mL, under orbital stirring at 170 rpm for 48 h. The reaction was conducted using 25g of oleic acid as the acyl donor group, 4.25g of the suspension of 1% m/m CNF in DMF, 33g of DMF, and 2g of lipase (CALB, Sigma). After 48h, the reaction mixture was centrifuged under the conditions mentioned above to remove residual organic acid, and the solid material (CNF) was resuspended in 10 mL of distilled water and heated to boiling for 5 min in order to deactivate the enzyme. Then, the suspension was centrifuged again and the obtained pellet was initially resuspended in 10 mL of acetone and centrifuged 4 times. After removing the supernatant in the fourth centrifugation step, the CNF was resuspended in 10 mL of 1% aqueous solution of sodium dodecyl sulfate (SDS) and centrifuged 5 times. Finally, the CNF obtained after the fifth centrifugation was resuspended in 10 mL of distilled water and centrifuged again. The CNF obtained after these steps was used for particle size analysis, atomic force microscopy - AFM, contact angle and X-ray excited photoelectron spectroscopy - XPS.

Contact angle measurements were performed using the Rame-Hart 300F1 goniometer. The equipment was used to measure the contact angle with drops of 1 µL of deionized water on a film prepared with CNF and functionalized CNF samples. The films were prepared by drying a diluted solution (0.1%) of CNF and functionalized CNF for 24 h at room temperature. To allow good statistical control of the measurement, 2 drops were placed in each sample and 5 angular measurements were taken in each drop. It is important to emphasize that it was not possible to quantify the contact angle value of the control due to its high hydrophilicity, that is, the value is 0 or close to 0. Initially, it is possible to notice that the biochemical route used for functionalization showed a positive result, when compared to non-functionalized CNF (control). Oleic and butanoic acids showed high hydrophobicity when compared to the control. It is speculated that the number of carbons present in its molecule, when reacting and forming a chemical bond with CNF, thus transfers its high hydrophobic character.

The surface modification of CNFs was further analyzed by X-ray photoelectron spectroscopy (XPS). The analysis was performed using a commercial spectrometer (Thermo Scientific K-Alpha) equipped with a Mg K line (h = 1253.6 eV) at low pressure and operated at 10 eV. When analyzing the proportions of the carbon atom before and after the functionalization, according to Table 3, below, an increase in the amount of carbon atoms present on the surface is verified, these atoms coming from the oleic acid linked to the CNF polymer. CNF isolated from Bleached Eucalyptus Kraft Pulp control (Figure 9A) and after enzymatic functionalization treatment (9B). Spectra were generated using the X-ray photoelectron spectroscopy (XPS) technique, using the premises of the National Nanotechnology Laboratory of the National Center for Research in Energy and Materials (CNPEM). In a practical way, it is possible to observe that there were changes in the connections present in the CNF. When comparing image 9A (control) and 9B (functionalized), there was an increase in the amount of carbon-carbon (C-C) bonds. Furthermore, when observing image 9B, the emergence of a new band (289.04 e. V) is verified, representing carbonyl-type bonds. Therefore, from the spectra generated, it is possible to prove, by chemical analysis, that there was a change in the chemical composition of the CNF surface, originating from the esterification reactions with oleic acid (an organic acid with a carbon chain of 18 carbons).

Table 4 presents the proportion of carbon atoms and their relation to the oxygen atoms found on the surface of films made from CNF derived from Bleached Kraft Pulp, before and after the enzymatic esterification reactions.

| **Sample** | **Carbon (%)** | **Nitrogen (%)** | **Oxygen/carbon ratio** |
|---|---|---|---|
| CNF BEKP | 60.2±0.1.53 | - | 0.62 |
| CNF BEKP FAO | 64.04±0.03 | 1.91±0.11 | 0.48 |

The present invention also provides the use of a lipase for the functionalization of a cellulose, in the proportion of lipase/cellulose being 47 g of lipase for each 1 g of cellulose. In this sense, Example 3 reveals the enzymatic functionalization of nanofibrillated cellulose isolated from bleached sugarcane bagasse pulp.

Initially, the cellulosic pulp of sugarcane bagasse used was generated after pre-treatment by steam explosion followed by alkaline delignification. The bleaching of the material resulting from the delignification process was carried out in two stages, one acidic and the other alkaline. In the first stage, in a reactor Parr com 2 L of capacity, 50 g of dry material were treated, with a solid-liquid ratio of 1:20. Hydrogen peroxide (H2O2) was added to a concentration of 8% (m/m), as well as 3% NaOH (m/m) and 1.2% MgSO₄ (m/m). The reaction took place for 2 h at 80 ± 2 °C, under constant agitation (120 rpm). After this step, the material was filtered and washed with a volume of distilled water proportional to 20 times the mass of the dry material, 10 times in a mixture and 10 times in a vacuum filtration flow. The second stage was carried out in the same reactor, with the same solids ratio (1:20), using all the mass recovered from the previous reaction, and adding concentrated acetic acid until reaching a concentration of 2.0% v/m (volume in relation to dry mass), as well as sodium chlorite 6% m/v. The reaction was carried out at 75 ± 2 °C for 2 h. In order to remove an even higher concentration of lignin, this step was repeated two more times. Therefore, 4 bleaching reactions were carried out, one in an acid medium and three in a basic medium.

Aiming at the production of nanofibrillated cellulose (NFC), a cellulose suspension (1% solids (m/m)) isolated from bleached sugarcane bagasse was processed in an ultra-disk refiner (SuperMassColloider, Masuko, Model MKCA6-5J), using water as solvent. The discs (model MKGA10-80) are made of ceramic material (aluminium oxide and resin) without porosity, preventing any nanometric particle infiltration and also allowing a better fit between the discs. One of the disks remained stationary and the other in motion, rotating at 1,600 rpm. The distance between the discs was determined and fixed. Briefly, the zero position of movement was determined by means of the noise generated by the disks touching each other. From the zero-movement position, the ultra-refiner was fed with the cellulose suspension and the discs were immediately adjusted to the position -100 µm of amplitude between discs. Although the position is negative, the presence of the cellulose suspension ensures that the discs do not touch each other and that the discs do not wear out.

CNF suspension sample taken right after processing (energy consumption of 40 kWh/kg) was diluted to a concentration of approximately 0.01% (m / v) and dripped onto the mica support. The mica with the samples was inserted into the desiccator for at least 4h. After sample preparation, images were taken using the Nanosurf FlexAFM microscope (Switzerland) and FMR silica probe (Nanoworld) at the National Nanotechnology Laboratory (LNnano) of the Surface Sciences Laboratory, National Center for Research in Energy and Matter (CNPEM, Campinas, Brazil). The system was operated using the intermittent contact technique, at a nominal resonant frequency of 75 kHz and a nominal force constant (spring) of 2.8 m/m. The results indicate that under the conditions used, it was possible to produce CNF with extremely small diameters and lengths greater than 1 µm (Figure 10). From the images treated in the Gwyddion 2.49 Software (64-bit) program, 100 diameter measurements of some CNF particles were taken and plotted in a graph in the Origin software (Version 2019) to analyze the diameter class and the average diameter. The values found indicate that most of the CNF produced has a diameter ranging between 10 and 20 nm, as can be seen in Figure 11.

Contact angle measurements were thus performed using the Rame-Hart 300F1 goniometer (Table 5). The equipment was used to measure the contact angle with drops of 1 µL of deionized water on a film prepared with CNF and functionalized CNF samples. The films were prepared by drying a diluted solution (0.1%) of CNF and functionalized CNF for 24 h at room temperature. To allow a good statistical control of the measurement, 3 drops were placed in each sample and 5 angular measurements were taken in each drop. It is important to emphasize that it was not possible to quantify the contact angle value of the control due to its high hydrophilicity, that is, the value is 0 or close to 0 (JONOOBI et al., 2010a; BOŽIČ et al., 2015). Initially, it is possible to notice that the biochemical route used for functionalization showed a positive result, when compared to non-functionalized CNF (control). Oleic and butanoic acids showed high hydrophobicity when compared to the control.

**Table 5: Values of contact angles of films produced with CNF (bleached sugarcane bagasse pulp) functionalize.**

| **Acyl donor group** | **Contact angle (°C)** |
|---|---|
| Control | 0 |
| Butanoic Acid (C4:0) | 84.5±01 |
| Oleic acid (C18:1) | 86.6±0.2 |

The surface modification of CNFs was further analyzed by X-ray photoelectron spectroscopy (XPS). The analysis was performed using a commercial spectrometer. (Thermo Scientific K-Alpha) equipped with Mg K line (h = 1253.6 eV) at low pressure and operated at 10 eV. The results indicate that there were clear changes in the chemical composition of the surface, increasing the number of carbon-carbon and oxygen-carbonyl bonds in the cellulose from different raw materials of plant origin, as can be seen from the comparison between Figures 12A and 12B, when analyzing the proportions of the carbon atom before and after the functionalization, according to Table 6, below, an increase in the amount of carbon atoms present on the surface is verified, these atoms coming from the oleic acid linked to the CNF polymer. Specifically, Figures 12A and 12B show Spectra of the bonds present in cellulose generated using the X-ray photoelectron spectroscopy (XPS) technique: CNF isolated from bleached sugarcane bagasse pulp control (Figure 12A) and after the enzyme functionalization treatment (Figure 12B). Spectra were generated using the X-ray photoelectron spectroscopy (XPS) technique, using the premises of the National Nanotechnology Laboratory of the National Center for Research in Energy and Materials (CNPEM). In a practical way, it is possible to observe that there were changes in the chemical bonds present in CNF. When comparing image 12A (control) and 12B (functionalized), there was an increase both in the amount of carbon-carbon (C-C) and carbonyl-type bonds (O-C=O). The raw material used to isolate the cellulose was bleached sugarcane bagasse, which has residual values of lignin (approximately 4%). This lignin present in CNF has carbonyls (ester groups), even in the control material. Therefore, from the generated spectra, it is possible to prove, by chemical analysis, that there was a change in the chemical composition of the CNF surface, originating from the esterification reactions with oleic acid (organic acid with 18 carbons).

Table 6 displays the proportion of carbon atoms and their relation to oxygen atoms found on the surface of films derived from CNF derived from sugarcane bagasse, before and after the enzymatic esterification reactions.

| **Sample** | **Carbon (%)** | **Nitrogen (%)** | **Oxygen/carbon ratio** |
|---|---|---|---|
| CNF BB | 57.69±0.57 | - | 0.73 |
| CNF BB FAO | 59.33±0.29 | 2.35±0.05 | 0.58 |

Analyzing the obtained results, it is possible to conclude that there were chemical and physical modifications on the surface of the cellulose used, both the BEKP derivative and the bleached bagasse, these modifications being the result of esterification reactions catalyzed by lipase, thus increasing its hydrophobicity. Unlike currently existing technologies, the proposed methodology aims to hydrophobize CNF using the lipase enzyme and use organic acids as acyl donor groups.

Therefore, it is demonstrated that the technology is functional regardless of the source of cellulose and the type of organic acid. In this sense, it corroborates Example 4, which illustrates an enzymatic functionalization reaction with immobilized enzyme.

Initially, the CMF solvent exchange was carried out in the same way as mentioned in the previous examples. The functionalization reactions with the immobilized enzyme (Lipase acrylic resin) were carried out in 50 mL Falcon-type centrifuge tubes, with a total reaction mass of 45.6g, being 6g of suspension of CMF close to 1% m/m, 32.5g of butanoic acid, 5.9g of DMF, 1.2g of enzyme under tumbling stirring at 170 rpm. The amount was 2g of enzyme in the first reaction. For the other reactions, the reaction values were adapted based on the amount of recovered enzyme, always maintaining the initial proportion. Parameters such as temperature and time, which were kept constant for all recycle reactions at 45 °C and 48 h, respectively.

For the recovery of the immobilized enzyme, a process of separation of the enzymes was used before (upstream) and after (downstream) using two filtration systems. Filtration System 1 uses a stainless-steel metal mesh filter with a porosity of 0.5 mm. Differently, Filtration System 2 uses a Buchner funnel combined with Whatman filter paper number 1. Initially, the enzyme was washed with exhausting water in Filtration System 1 and later washed with hexane in Filtration System 2. Both coupled to a vacuum system. After these steps, the enzyme was dried in an oven (45°C) and stored at 3°C until its addition to the reaction medium (upstream step). At the end of each reaction (downstream step) the reaction medium was placed in Filtration System 1 and 200 ml of distilled water were added. The filter cake was then removed and placed on filtration system 2 and hexane was added. This operation was repeated two more times, and the filtered material was then centrifuged at 4,789g for 5 min to remove butyric acid and DMF and consequent concentration of CMF. At the end of the separation steps, the recovered immobilized enzyme was stored in a refrigerator. The CMF from all separation steps was centrifuged in aqueous medium three more times or until the variation in the pH value ceased, indicating that the excess acid was removed.

Using the AFM-FTIR technique, it is possible to verify the presence or absence of the C-O-OH (carboxyl) bands of esters, found in the region from 1730 to 1750 cm⁻¹. In addition, typical bands of O-H bonds present in cellulose found in glucose monomers can also be seen (1640 cm⁻¹), as shown in Figure 13. In the obtained spectra, the functionalized CMF (CMF-Fs) showed the C-O-OH bands (carboxyl) not evidenced in the control CMF, chemically indicating the esterification of the microfibers. Therefore, it was possible to chemically prove the occurrence of CMF functionalization in three different recycle reactions, demonstrating that it is possible to esterify CMF using the same enzyme for at least five times (CMF-F5). The results demonstrated here do not indicate the intensity of the functionalization due to the fact that this technique is only qualitative.

Contact angle values for functionalized CMF films (CMF-Fs) with immobilized lipase are shown in Table 7 below. The CMF film functionalized with immobilized lipase in the first reaction (CMF-F1) resulted in a contact angle of 114°. Furthermore, the immobilized enzyme was able to functionalize up to the fifth recycle reaction. Therefore, the contact angle values obtained were higher than the control (CMF), demonstrating that the CMF was functionalized in all five recycle reactions tested.

Table 7 displays the contact angle for CMFs functionalized using immobilized enzyme.

| **Recycle** | **Sample-Reaction** | **Contact angle (θ)** |
|---|---|---|
| - | CMF (control) | 30° |
| - | CMF-F1 | 114.9 ° |
| 1 | CMF-F2 | 77.2 ° |
| 2 | CMF-F3 | 66.7 ° |
| 3 | CMF-F4 | 55.4 ° |
| 4 | CMF-F5 | 74.5° |

| | | |
|---|---|---|
| *CMF: CMF control ** CMF F1: CMF after one reaction with the immobilized enzyme, CMF-F2: CMF after two reactions with the same immobilized enzyme used before and therefore up to CMF-5. | | |

Once again, it is demonstrated that the present invention is functional independent of the source of cellulose and the type of organic acid using free and immobilized lipase.

Functionalized cellulose, object of the present invention, can also be incorporated into an article for use, such as films, non-aqueous formulations based on organic solvents, which allows obtaining an article comprising functionalized cellulose, in which it presents a degree of functionalization (replacement) of at least 0.01.

The proposed technology has numerous applications and can generate anything from a new material with high added value to being used to reduce costs for pulp transport, especially at micro and nano scales, reducing the previously existing hornification process, such as the agglomeration of cellulose micro and nanofibrils due to the high number of hydroxyls present on the surface during the drying process, being a negative effect reducing its technical properties. Therefore, the hydrophobic functionalization process catalyzed by enzymes, particularly lipases, using organic acids, preferably an organic acid with a carbon chain of 2 to 18 carbons, more preferably fatty acids and, even more preferably, fatty acids selected from butanoic acid, oleic acid, palmitic acid, myristic acid or mixtures thereof; has attractive characteristics, such as mild reaction conditions and is easily applicable in several industrial sectors, including the paper, packaging and biomedical sectors. Such celluloses with increased hydrophobicity have as a differential the high hydrophobicity with greater compatibility with a wide range of materials, in addition to applying, as raw material, an industrial by-product, extremely abundant.

## Claims

1. FUNCTIONALIZED CELLULOSE, **characterized in that** the cellulose comprises hydrophobic ester groups.

2. FUNCTIONALIZED CELLULOSE, according to claim 1, **characterized in that** the cellulose has a degree of substitution of at least 0.01.

3. FUNCTIONALIZED CELLULOSE, according to the previous claims, **characterized in that** the cellulose can be a cellulose fiber, a cellulose microfibril, a microfibrillated cellulose, a cellulose nanofibril, a nanofibrillated cellulose, a cellulose filament, or mixtures thereof.

4. FUNCTIONALIZED CELLULOSE, according to claim 1, **characterized in that** the hydrophobic ester groups come from organic acids, preferably organic acids with a carbon chain of 2 to 18 carbons.

5. FUNCTIONALIZED CELLULOSE, according to claim 1, **characterized in that** the organic acid is of vegetable origin.

6. FUNCTIONALIZED CELLULOSE, according to claims 4 or 5, **characterized in that** the organic acid is selected from oleic acid, linoleic acid, linolenic acid, butanoic acid, palmitic acid, myristic acid, caprylic acid, capric acid, lauric acid, stearic acid, melissa, valeric acid, or mixtures thereof.

7. FUNCTIONALIZED CELLULOSE, according to claims 1 to 6, **characterized in that** the enzyme is a lipase.

8. FUNCTIONALIZED CELLULOSE, according to claim 7, **characterized in that** the lipase is selected from: Amano Lipase A, Lipase from porcine pancreas, Lipase from *Candida sp, antartica* and *rugosa,* Lipase from *Aspergillus oryzae,* Lipase B *Candida antarctica* immobilized on Immobead 150.

9. METHOD OF ENZYME FUNCTIONALIZATION OF CELLULOSE, **characterized by** the method comprising the introduction of ester groups from organic acid into cellulose by the action of an enzyme.

10. METHOD OF ENZYME FUNCTIONALIZATION OF CELLULOSE, according to claim 10, **characterized in that** the method comprises the functionalization of each 1 g of cellulose with at least 1 M of organic acid in the presence of at least 1 g of enzyme.

11. METHOD OF ENZYME FUNCTIONALIZATION OF CELLULOSE, according to claim 10, **characterized in that** the method comprises the functionalization at a cellulose: organic acid: enzyme ratio of 1:50:1.

12. METHOD OF ENZYME FUNCTIONALIZATION OF CELLULOSE, according to claims 9 to 11, **characterized in that** the method comprises the functionalization for each 1 g of cellulose by 208g to 416.66g of butanoic acid in the presence of at least 7.43g of lipase.

13. METHOD OF ESTERIFICATION OF CELLULOSE WITH ORGANIC ACID, **characterized by** the introduction of ester groups from organic acid in cellulose by the enzymatic catalysis of a lipase.

14. ENZYME FUNCTIONALIZATION PROCESS OF CELLULOSE FROM AN ORGANIC ACID, **characterized by** comprising the following steps:
1. Dispersing a cellulose in an aqueous medium;
2. Replacing the aqueous medium with an ethanol/acetone mixture;
3. Resuspending the cellulose in dimethylformamide, obtaining a mass concentration of at least 1%;
4. Keeping under agitation, adding, for each 100g of cellulose suspension at 1% m/m DMF, an acid: enzyme mass ratio of at least 50:1, producing a reaction mixture, then letting it rest;
5. Recovering a functionalized cellulose by centrifuging the suspension;
6. Optionally, resuspending the functionalized cellulose in acetone, followed by centrifugation, removing the supernatant and resuspending the cellulose in an aqueous solution of 1% sodium dodecyl sulfate (SDS),
7. Optionally, followed by centrifugation to obtain functionalized cellulose for resuspension in distilled water.

15. ENZYME FUNCTIONALIZATION PROCESS OF CELLULOSE FROM AN ORGANIC ACID, according to claim 14, **characterized in that** step 4 comprises at least 221g of DMF.

16. ENZYME FUNCTIONALIZATION PROCESS OF CELLULOSE FROM AN ORGANIC ACID, according to claim 14, **characterized by** the step (1) of cellulose dispersion in aqueous medium at a proportion of at least 0.25% by mass of cellulose.

17. ENZYMATIC FUNCTIONALIZATION PROCESS OF CELLULOSE FROM AN ORGANIC ACID, according to claim 14, **characterized by** the ethanol/acetone mixture of step (2) being in a ratio of 1:5 to 5:1.

18. ENZYME FUNCTIONALIZATION PROCESS OF CELLULOSE FROM AN ORGANIC ACID, according to claim 14, **characterized by** the rest of step (4) being at least 12 h, preferably 72 h.

19. ENZYME FUNCTIONALIZATION PROCESS OF CELLULOSE FROM AN ORGANIC ACID, according to claim 14, **characterized in that** step (4) is followed by a step (4a) in which the reaction mixture is centrifuged to remove residual organic acid.

20. ENZYME FUNCTIONALIZATION PROCESS OF CELLULOSE FROM AN ORGANIC ACID, according to claim 14, **characterized in that** step (4a) is followed by a step (4b) in which the enzyme is deactivated by heating.

21. METHOD FOR THE PRODUCTION OF A PULP WITH INCREASED HYDROFOBICITY, **characterized by** comprising:
(a) supplying a cellulosic feedstock;
(b) in the presence of a lipase, functionalizing said cellulosic raw material, by introducing ester groups derived from an organic acid, to generate a functionalized cellulose with increased hydrophobicity; and
(c) recovering said functionalized cellulose with increased hydrophobicity.

22. USE OF A LIPASE FOR THE FUNCTIONALIZATION OF A CELLULOSE, according to claim 21, **characterized in that** the proportion of lipase/cellulose is at least 1 g of lipase for each 1 g of cellulose.

23. ARTICLE INCLUDING FUNCTIONALIZED CELLULOSE, according to claim 21, **characterized in that** cellulose has a degree of functionalization with a degree of substitution of at least 0.01.
